# EUROPEAN PATENT SPECIFICATION

(11) **EP 3 979 966 B1**
(45) Date of publication and mention of the grant of the patent: **21.01.2026**
(21) Application number: 20817912.7
(22) Date of filing: 04.06.2020
(51) Int. Cl.: A61H 1/02, A63B 23/12, G06F 3/00

(54) **A SELF DRIVEN REHABILITATION DEVICE AND METHOD THEREOF**
SELBSTANGETRIEBENE REHABILITATIONSVORRICHTUNG UND VERFAHREN DAFÜR
DISPOSITIF DE RÉÉDUCATION AUTONOME ET PROCÉDÉ ASSOCIÉ

(30) Priority: 07.06.2019 IN 201941022639
(43) Date of publication of application: 13.04.2022
(73) Proprietor: Osind Medi Tech Private Limited, Manipal Udupi, Karnataka 576104 (IN)
(72) Inventor: VENKATESAN, Venkateswara Subramania, Manipal Karnataka 576104 (IN); SAMHEEL, Mohammad, Brahmavar Udupi, Karnataka 576213 (IN); BHAT, Anil K, Manipal Karnataka 576104 (IN)
(74) Representative: Wetzel, Fritz
(86) International application number: PCT/IB2020/055273
(87) International publication number: WO 2020/245762

(56) References cited:
- CN-A- 107 242 958
- CN-A- 107 242 958
- US-A1- 2012 157 263
- US-A1- 2014 142 470
- US-A1- 2015 328 492
- US-A1- 2016 259 417
- US-A1- 2017 165 567
- US-A1- 2018 098 583
- US-A1- 2018 335 841
- US-B2- 9 326 909
- US-B2- 9 326 909
- YAP HONG KAI, KHIN PHONE MAY, KOH TZE HUI, SUN YI, LIANG XINQUAN, LIM JEONG HOON, YEOW CHEN-HUA: "A Fully Fabric-Based Bidirectional Soft Robotic Glove for Assistance and Rehabilitation of Hand Impaired Patients", IEEE ROBOTICS AND AUTOMATION LETTERS, vol. 2, no. 3, 15 February 2017 (2017-02-15), pages 1383 - 1390, XP055766653

## Description

### Field of the Invention:

The present invention provides a self- driven Rehabilitation Device and its method of operation, useful in stroke and neuro-muscular deficit patients.

### Background of the Invention:

The following background discussion includes information that may be useful in understanding the present invention.

Stroke victims often lose proper function of at least one hand and fingers, experiencing delays in gripping and releasing ability, according to Science Daily. The symptoms arise from the effects of strokes on fine motor control in the brain, which regulates movement through muscles, the skeleton and neurological messages, the American Heart Association reports. Strokes often cause at least temporary paralysis on one side of the body, including hands and fingers. The side of the body affected by strokes depends on the side of the brain in which strokes occur, with left brain strokes affecting the right side of the body and right brain strokes affecting the left side, according to the Brain Foundation.

The literature states that Hand and finger exercises, movement and physical therapy help stroke victims relearn the use of fine motor skills.

CN102387760B discloses kind of wearable action-assist device comprising motion assisting glove (20) having a wearer's finger inserted into the finger insertion portion (21), a drive unit (40), arranged in the auxiliary operation glove back (20) side of the driving finger inserting portion (21), a linear member (50), inserted along the portion of the finger (21) extending in the direction of the drive unit is configured to the drive force to the finger insertion section (21), biological signal detecting unit (60), for detecting the finger movement of the wearer's biosignal, a control unit (70).

US20190021929 discloses a method of rehabilitation using an actuator type that includes a movement mechanism capable of applying a force that interacts with a motion of a patient's limb in a volume of at least 30 cm in diameter, in at least three degrees of freedom of motion of the actuator and capable of preventing substantial motion in any point in any direction in said volume, in which a same movement mechanism is used at two different places of rehabilitation.

US2019105524 discloses a rehabilitation and mobility improvement apparatus **(RMIA)** comprising a garment, a flexible connection member and an appendage support device, wherein said flexible connection member is connected to said garment on a first end and is connected to said appendage support device on a second end.

US 9,326,909 discloses a therapeutic device for improving voluntary control of paretic muscles in a patient extremity. The therapeutic device is designed to be portable and may be strapped onto a patient' s wrist or ankle. The device employs a plurality (Continued) of micro-motors configured to deliver vibratory sensations to a patient extremity as somatosensory inputs. Each micro motor is dimensioned to reside on a patient's respective finger or along their foot. However, US '909 fails to provide the feature of mobilization. It delivers vibratory sensations to a patient extremity but no actual movement is being performed.

CN105496728A discloses a soft-bodied robot glove for hand movement function recovery. The soft-bodied robot glove comprises a decision processing module, a driving module, a flexible exoskeleton glove and a Bowden cable. The decision processing module is electrically connected with the driving module. One end of the Bowden cable is connected with the finger end of the flexible exoskeleton glove, and the other end of the Bowden cable is connected to a winch of the driving module. However, CN '728 fails to disclose a modular connection which could help user to select the finger to be mobilized. The system disclosed by CN '728 is highly complex and not portable. It uses the driving module arranged on the single line.

US 2014/142470 A1 relates to a rehabilitation apparatus. More specifically it relates to an apparatus for rehabilitation of a person who has suffered traumatic injury more, specifically a stroke. US 2014/142470 A1 does not disclose that a plurality of metal hooks connected to a rhodium plated column comprising a barrel swivel clasps/connectors wherein the barrel swivel connectors connect the glove to a motorized unit.

The conventional devices are normally based on passive mobilization therapy and are not the self-driven devices with real-time mirroring effect for better/enhanced visual feedback. The present Invention addresses this technical problem and provides a highly efficient self-driven rehabilitation device and method thereof.

### Objective of the Invention:

The primary object of the present invention is to overcome the drawback associated with prior art as recited in claim 1.

The present invention therefore provides a self-driven Rehabilitation Device useful in stroke and neuro-muscular deficit patients.

Yet another object of the present invention is to provide a self-driven Rehabilitation device with real-time mirroring effect for better visual feedback as compared to the existing devices which are normally based on passive mobilization therapy.

Yet another object of the present invention is to provide an automatic, portable, customizable, compact, light-weight, portable, easy to use self-driven Rehabilitation Device.

Yet another object of the present invention is to provide a wearable, highly efficient and cost effective self-driven Rehabilitation Device.

### Summarv of the Invention:

In an aspect of the Invention, there is provided a customizable and light-weight self-driven rehabilitation device with real time mirroring effect for mobilizing an affected or paralyzed hand of a user comprising:
a pair of gloves comprising at least a sensor glove, adapted to be worn in the normal functioning hand of the user, and a modular motorized glove, adapted to be worn in said paralyzed hand of the user,
wherein said sensor glove is connected with the modular motorized glove through a communication module for active and/or passive mobilization of the paralyzed hand through real time mirroring effect,
wherein sensor glove, provided with an easy to slide system for the fingers, comprises:
   A. at least a sensing module comprising plurality of flex sensors for sensing movement of fingers based on the change in resistance;
   B. a control/ circuit box comprising a communication module for collecting the data from sensing module and transmitting to the motorized glove;
   C. a clincher connector/s connects the sensing module with the control/circuit box; and
   D. a filter circuit adapted remove the noise signal between the sensing module and control box;
   E. a powered supply means comprising a battery for powering the sensor module and control module;
wherein the modular motorized glove, is provided with a drive mechanism adapted to be positioned on the arm of a user, comprises:
   A. plurality of fingertip caps, configured to be worn on to the finger tips, attached to a tensioning string;
   B. motorized unit, configured to be placed in the arm of user, comprising:
      i) plurality of motors for allowing the independent control of the fingers of the paralyzed hand wherein each motor is connected to individual strings for extension;
      ii)a motor driver unit or mechanism to drive said plurality of motors wherein the driver unit or mechanism is adapted to move towards back on the arm for increased range of motion; wherein the motor driver unit and mechanism comprises 2 motor or 5 motor operation system, wherein the 2 motor operation system operates in plurality of active and/or passive mode by connecting at least two fingers of the hand or its combination thereof while the 5 motor operation system operates in plurality of modes based on the requirement of mobilization wherein the 5 motor operation system allows independent movement of each finger facilitating plurality of modes of operation for active and/or passive mode;
      iii) a microcontroller unit with communication module configured to receive the data transmitted from said sensor glove to accordingly control the said motorized unit;
         wherein the strings are connected through the modular connector acting like a bridge between the motorized unit and glove;
      iv) a power supply means to power the motor driver unit and the microcontroller unit;
      vi) at least a closure unit or mechanism being placed at the top of said motorized unit with a bridge in between, said closure unit or mechanism comprises the characterizing feature of a dial which allows the torque to be adjusted by its rotation for obtaining range of mobilization;
      vii) connecting means comprising modular connector for selecting the finger to be mobilized and for the activation of active and/or passive mode of therapy based on requirement;
where said device optionally comprises a wrist splint for restricting the wrist movement . characterized in that the modular motorized glove comprises a plurality of metal hooks connected to a rhodium plated column comprising barrel swivel clasp connectors wherein the barrel swivel connectors connect the glove to the motorized unit.

### Detailed Description of Drawings:

To further clarify advantages and features of the present invention, a more particular description of the invention will be rendered by reference to specific embodiments thereof, which is illustrated in the appended drawings. It is appreciated that these drawings depict only typical embodiments of the invention. The invention will be described and explained with additional specificity and
detail with the accompanying drawings in which:
**Figure 1****:** illustrates the functioning of the device according to an embodiment of the invention
**Figure 2****:** illustrates (a) the front side of the sensor glove and (b) the back side of the sensor glove
**Figure 3****:** illustrates the inner layer of the sensor glove enclosing sensor circuit box and sensor glove
**Figure 4****:** illustrates components of the device according to an embodiment of the invention
**Figure 5****:** illustrates components of the sensor glove- 5(a) illustrates flex sensors; 5(b) illustrates clincher connectors; 5(c) illustrates filter circuit; 5(d) illustrates BLE module or wi-fi module; 5(e) illustrates battery powering the sensor module; 5(f) illustrates sensor glove circuit box
**Figure 6****:** illustrates the casing design within glove as illustrated in figure 6(a), Casing within sensor module in figure 6(b), Top view of charging port on and off button in figure 6 (c), Side view of charging port ON and OFF button in figure 6 (d)
**Figure 7****:** illustrates the front side of the motorized glove
**Figure 8****:** illustrates the inside layer front of the motorized glove where 1 cm strips is stitched on the edge to cover metal string from inside
**Figure 9****:** illustrates the back side of the motorized glove showing tensioner location and connections
**Figure 10****:** illustrates functioning of a two-motor device and a five-motor device in accordance with an embodiment of the Invention.
**Figure 11****:** illustrates the two-motor device having modular nature
**Figure 12****:** illustrates functioning of the five-motor device
**Figure 13****:** illustrates an embodiment showing base of the closure unit or mechanism, as provided by the present invention
**Figure 14****:** illustrates another embodiment showing top of the closure unit or mechanism, as provided by the present invention
**Figure 15****:** illustrates another embodiment showing the closure system bridge servo horn attachment

### Detailed Description:

For the purpose of promoting an understanding of the principles of the invention, reference will now be made to the embodiment illustrated in the drawings and specific language will be used to describe the same. It will nevertheless be understood that such alterations and further modifications in the illustrated system, and such further applications of the principles of the invention as illustrated therein being contemplated as would normally occur to one skilled in the art to which the invention
relates.

It will be understood by those skilled in the art that the foregoing general description and the following detailed description are exemplary and explanatory of the invention and are not intended to be restrictive thereof.

The Invention provides a self-driven Rehabilitation Device or system useful in stroke and neuro- muscular deficit patients and its method of operation.

The device or system is a self-driven Rehabilitation device with real-time mirroring effect for better visual feedback as compared to the existing devices which are normally based on passive mobilization therapy. The device or system allows smooth and realistic movements. The device or system is helpful for the patients who cannot do regular therapy due to distance between their home and the hospital with a home-based rehabilitation program.

The present device or system has various technical advantages in terms of being automatic, portable, customizable and compact. The device or system is cost effective, light weight, portable, easy to use and customizable to fit as needed. The system is wearable and highly portable. This allows the therapy to be done from home without the supervision of the doctor or therapist. The user movements can be tracked using remote monitoring for gathering data of usage.

**In** an embodiment, the customizable and light-weight self-driven rehabilitation device with real-time mirroring effect for mobilizing an affected or paralyzed hand of a user comprises a pair of gloves comprising at least a sensor glove, adapted to be worn in the normal functioning hand of the user, and a modular motorized glove, adapted to be worn in said paralyzed hand of the user. The sensor glove is connected with the modular motorized glove through a communication module for active and/or passive mobilization of the paralyzed hand through real time mirroring effect. The sensor glove is provided with an easy to slide system

**In** an embodiment, the sensor glove comprises following components:
1. At least a sensing module comprising plurality of flex sensors for sensing movement of fingers based on the change in resistance;
11. a control or circuit box comprising a communication module for collecting the data from sensing module and transmitting to the motorized glove;
111. a Clincher connector connects the sensing module with the control/circuit box; and
1v. a filter Circuit adapted to remove the noise signal between the sensing module and control box;
v. a powered supply means comprising a battery for powering the sensor module and control module,

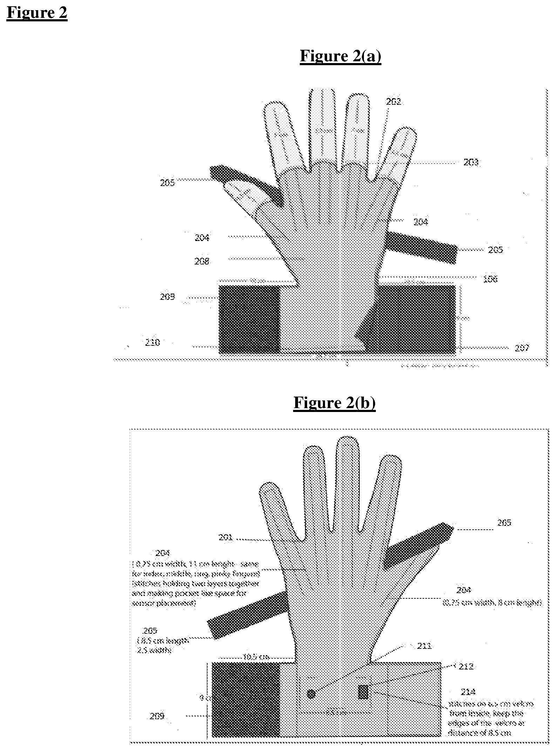

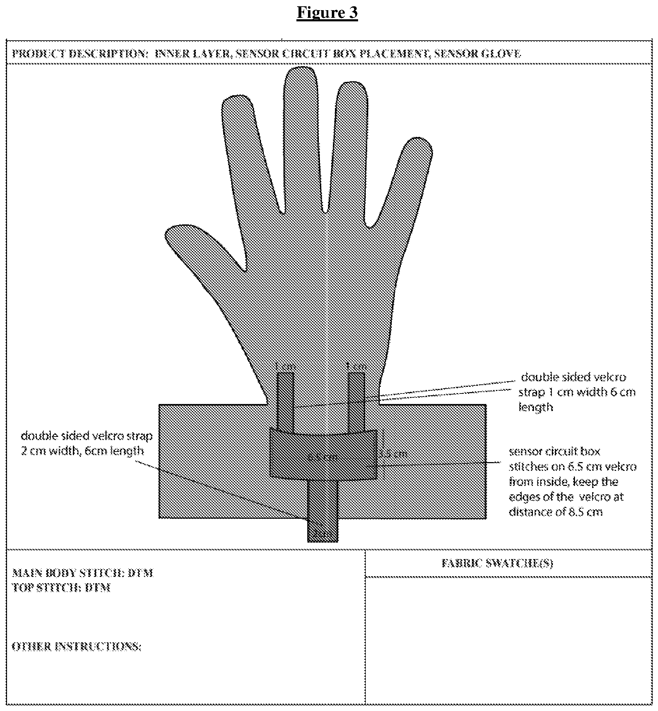

**In** an embodiment, the sensor glove comprises at least 3 layers. The first layer is illustrated in figure 2 which consists of part that comes in contact with the hand i.e. dorsal region of palm of the subject. The first layer consists of plurality of Velcro strips for binding the gloves and components that come in contact with the hand of the Subject or user The intermediate layer is illustrated in figure 3 which houses the sensor module (control box) while the third layer as illustrated in Figure 2a is configured to position the sensor module and the clincher connectors i.e. the sensor module groves are present for placement and clincher connectors are placed and connected.

The sensor glove uses an easy to slide system for the fingers with lesser cloth material on the palm. The control system is housed inside the glove itself as a sensor box with battery side for easy to wear. The sensors and its wiring are hidden within in the intermediate layer consisting of clincher connector for modular connectivity of sensors.

**In** an embodiment, the modular motorized glove is provided with a drive mechanism wherein the drive mechanism rests or is positioned on the arm of a user. Particularly, the motorized glove is worn on the hand and the drive mechanism is placed on the arm. Battery used is a powerbank which is placed nearby or in packet.

When in air, or surface, the load will applied on the fingers will be only of the glove which is approximately 50g. Hence, the device is super lightweight in comparison to the existing products in the market or known otherwise.

The modular motorized glove comprises following components:
i. plurality of fingertip caps, configured to be worn on to the finger tips, attached to a tensioning string;
ii. motorized unit, configured to be placed in the arm of user, comprising:
   a. plurality of motors for allowing the independent control of the finger/s of the paralyzed hand wherein each motor is connected to individual strings for extension;
   b. a motor driver unit/mechanism to drive said plurality of motors wherein the driver unit/mechanism is adapted to move towards back on the arm for increased range of motion;
   c. a microcontroller unit with communication module configured to receive the data transmitted from said sensor glove to accordingly control the said motorized unit;
      wherein the strings are connected through the modular connector acting like a bridge between the motorized unit and glove;
   d. a power supply means to power the motor driver unit and the microcontroller unit;
   e. plurality of metal hooks connected to a rhodium plated column comprising barrel swivel clasps or connectors wherein the barrel swivel connectors connect the glove to the motorized unit;
   f. at least a closure unit/ mechanism being placed at the top of said motorized unit with a bridge in between, said closure unit/mechanism comprises the characterizing feature of a dial which allows the torque to be adjusted by its rotation for obtaining range of mobilization;
iii. connecting means comprising modular connector for selecting the finger to be mobilized and for the activation of active and/or passive mode of therapy based on requirement.

The system optionally comprises a wrist splint for restricting the wrist movement.

Further, the device comprises the motor driver unit based ona 2-motor or 5-motor operation system. The 2-motor operation system operates in plurality of active and/or passive mode by connecting at least two fingers of the hand or its combination thereof. The 5-motor operation system operates in plurality of modes based on the requirement of mobilization. The 5-motor operation system allows independent movement of each finger facilitating plurality of modes of operation for active and/or passive mode.

In an embodiment, the motor operation system comprises a servo motor system.

In an embodiment, the bridge in the closure unit comprises a servo horn attachment. The closure unit/mechanism comprises a dial at the top. The dial allows the torque to be adjusted by its rotation and helps in setting the appropriate range of motion.

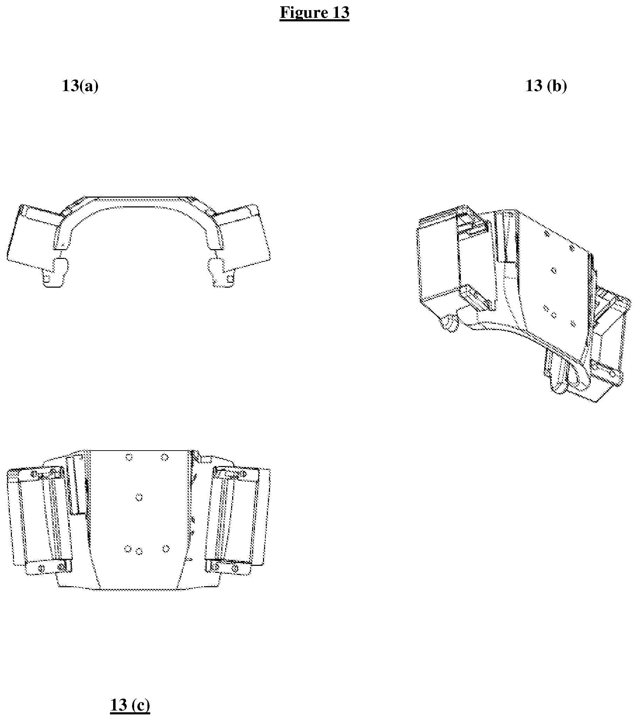

In an embodiment, **Figure 13** shows the base of the motorized unit with two-motor systems. The base of the motorized unit is configured to be placed or worn on the arm of the paralyzed hand or therapeutic hand for mobilization. Figure 13(a), 13(b) and 13(c) shows the front, side and top view of the base of said motorized unit. The motorized unit can easily be placed on the arm or rest on any surface without affecting the motion.

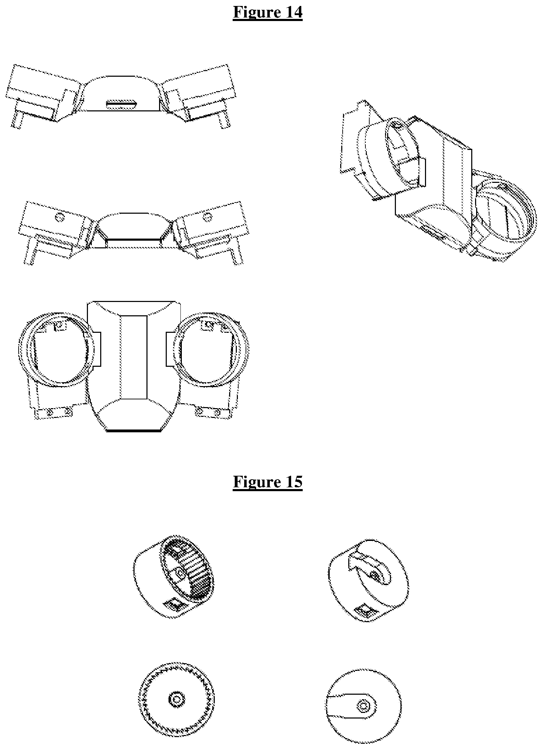

**Figure 14** shows the top of the motorized unit with two-motor systems which is configured to be placed or fitted with the bottom of the motorized unit. The top is adapted to house 2 motors, thus making it highly portable and easy to wear. The motor system simply runs with the power bank. The top of the motorized unit comprises a space for adapting a closure system bridge with servo horn attachment as shown in figure 15. This closure system comprises a dial at the top which allows the torque to be adjusted by rotating the dial and set appropriate

In an embodiment, the sensor glove comprises a plurality of layers wherein the layers comprise:
a) a first layer, configured to consist a plurality of Velcro strips for binding the gloves and components that come in contact with the hand of the subject/user;
b) an intermediate layer configured to in-house the sensor and connecting means comprising clincher connector; and
c) a third layer configured to position the sensor module and the clincher connectors.

In an embodiment, the system consists of two variable changes, as mentioned below, that account for change in range of mobilization.
a) Moving the drive mechanism further back on the arm for increased range; and
b) The Closure system dial which allows the torque to be adjusted by rotating the dial and setting appropriate range of mobilization. This allows the position of the drive mechanism to be unchanged and still benefit from varying the range of mobilization and torque. Both Palm surfaces are free for movement and can move the wrist. If the wrist movement needs to be restricted, the design is made in such a way that it can accommodate a wrist splint under the glove and drive mechanism, to allow maximum finger movement only.

In an embodiment, the plurality of motor comprises two motors for activating at least two fingers or a five-motor system for activating all the five fingers, as required.

In an embodiment, the sensor glove being in communication to the motorized glove provides feedback based on virtual reality environment.

In an embodiment, the sensor glove functions in modes comprising:
a. active mode for transmitting position data for mobilizing the finger/s;
b. diagnostic mode for wirelessly tracking the range of motion through a software application;
c. virtual reality gaming mode where the sensor glove tracks the range of motion and wirelessly transmits the data to a software application. The sensor glove is used for Non-Immersive or Immersive VR Gaming as a tool for motivation for people who have regained some motion but have to repeat it to keep the functional recovery. The sensor glove has the ability to track the movement (range of motion) as well as it wirelessly transmits data to android app and at the same time acts as controller for the game in Real Time.

In an embodiment, the glove comprises grooves for the strings to slide through with ease for providing maximum torque.

In an embodiment, the range of motion of the affected limb is remotely monitored by an application software for evaluating the improvement in the user.

In an embodiment, the clincher connectors comprise a plastic coating for eliminating the overlap of signals received from the plurality of sensors.

In an embodiment, the modular connector of motorized glove is in connection to the flexible fingertip cap where the fingertip cap also attaches itself to the tensioning system. on the back side to provide passive extension or passive flexion.

In an embodiment, the sensor glove comprises a force sensitive resistor on the fingertip to measure the pinch force for evaluating the improvement in the patient.

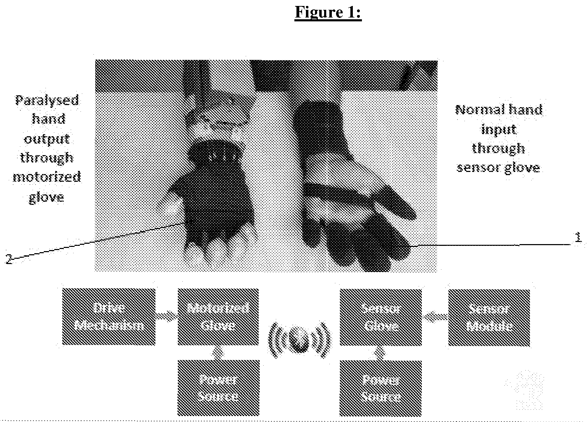

In an embodiment referring to the figure 1, the self-driven rehabilitation system comprises a pair of gloves having at least a motorized glove (2) and a sensor glove (1) along with a motorized unit (3).

In an embodiment, the sensor glove (1) is worn on the normal hand and the motorized glove (2) on the affected/ paralyzed hand and the motorized unit (3) is worn on the affected arm. The system is effective for mobilization of the hand and allows user to mirror the movements of his normal hand to the affected arm under active mode / active mobilization.

In an embodiment, the movements can be pre-programmed based on the users need under passive mode / passive mobilization.

In an embodiment, the sensor glove (1) communicates with the motorized glove (2) through communications module like blue tooth to replicate or mirror the activities done on the functional hand.

In an embodiment, the connecting mechanism between said modular and sensor glove is modular.

In an exemplary embodiment, Figure 2 (a) and 2(b) illustrates front side and back side of the sensor glove respectively.

Figure 2(a) shows front side of the sensor glove showing following components:
a) Multiple Velcro straps (205,209) having length ranging from 7.5 cm to 9 cm length and width ranging from 2.5 cm to 5 cm;
b) Edge stitch (201) in between the joint of fingers;
c) Multiple Sensors (204) having approximately 0.75 cm width and length ranging from 8 cm to 11 cm
d) Multiple layers (207,208) i.e. Layer 1 and Layer 2
e) Invisible zip(206)
f) Stitched portion (203) holding two layers together and making packet like space for sensor placement
g) Single fold stitch (210) having 1 cm length.

Figure 2 (b) shows back side of the sensor glove showing following components:
a) Multiple Velcro straps (205,209), having length ranging from 7.5 to 9 cm length and width ranging from 2.5 cm to 9 cm;
b) Edge stitch (203) in between the joint of fingers;
c) Multiple Sensors (204) having approximately 0.75 cm width and length ranging from 8 cm to 11 cm
d) at least a button hole (211) stitch- opening for on/off button
e) at least a button hole stitch- opening for charging port (212)
f) sensor circuit box (214) stitches on 6.5 cm Velcro from inside keeping the edges of the Velcro at distance of 8.5cm.

In an embodiment, the inner layer of the sensor glove is illustrated in **figure** 3. The inner layer comprises a double-sided Velcro strap (1 cm width and 6cm length), another double-sided Velcro (double-sided width with 6 cm length), sensor circuit box stitched on 6.5 cm Velcro from inside keeping the edges of the Velcro at distance 8.5 cm.

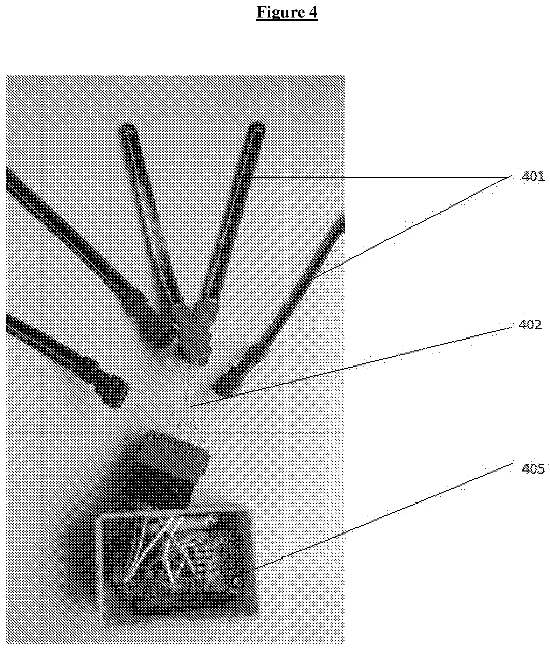

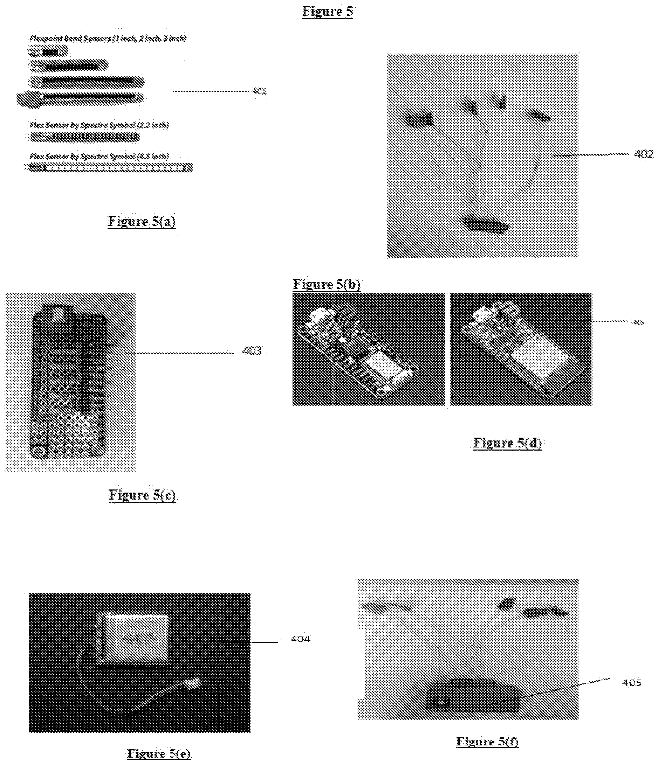

**Figure 4 to 5** shows comprehensive view of the components of the sensor glove. In an embodiment, the sensor glove comprises following components:
a) Flex Sensors (401)
b) Clincher connectors (402)
c) Filter Circuit (403)
d) Battery for powering the sensor module (BLE module or wi-fi module) (404)
e) Sensor Glove Circuit Box (405)

In an embodiment, the Flex Sensors (401) senses the movement of fingers based on the change in resistance. The flex sensors comprises flexpoint bend sensors (1inch, 2 inch, 3inch), flex sensor by spectra symbol (2.2 inch), flex sensor by spectra symbol (4.5inch). 1 inch corresponds to 2,54 cm.

In an embodiment, the clincher connectors are used for connection between the Flex Sensors and the Sensor Circuit. The wires connecting these two have a coating of plastic which doesn't allow overlap of the signals. The connection is modular using the clasps connector system. User can choose which finger to mobilize while connecting the clasps. The connecting mechanism is modular where the strings are connected to the Flexible fingertip cap where the Fingertip cap also attaches itself to the tensioning system on the back side to provide extension.

In an embodiment, the filter circuit filters the noise from the signal and provides the location for Switch ON/Off Button. It also acts as modular connecting point for clincher connectors. The filter circuit is based on the microcontroller / Communication channel used.

In an embodiment, the sensor glove comprises Adafruit NRF 51 (BLE module) or ESP 32 (Wi-Fi module). It further comprises microcontroller charging port and communication channel.

In an embodiment, the battery is Li po battery.

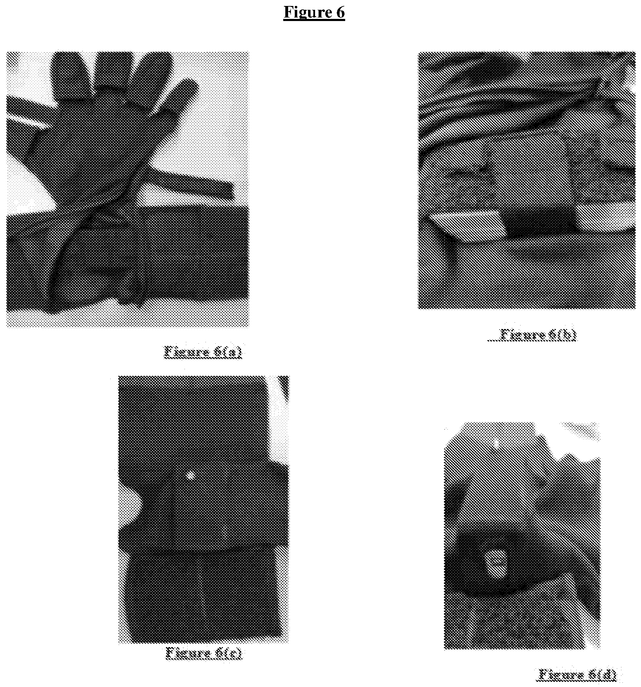

In an embodiment, the casing design within glove is illustrated in **figure** 6(a), Casing within sensor module is illustrated in **figure** 6(b), Top view of charging port on and off button is illustrated in figure 6 (c), Side view of charging port on and off button is illustrated in **figure 6 (d)**.

In an embodiment, the sensor glove provides a way to be used in Virtual Reality (Both Immersive and Non - Immersive environment) based rehabilitation using games for motivation for repetitive therapy. This allows the patient to use the sensor glove on normal hand and mobilize affected hand while playing a game or it can be used in strake patients who have partial movement but need motivation to do therapy.

In an embodiment, the sensor glove is combined with motorized glove to give the feedback based on virtual reality environment. As an example: If a user is trying to squeeze a lemon in virtual reality, then the sensor glove senses the movement and feeds the information to allow the motors to simultaneously produce proportional force to be applied in the opposite direction to get the haptic feedback.

The user is able to perform the accurate movement with the help of sensor glove by controlling the movement of the affected arm.

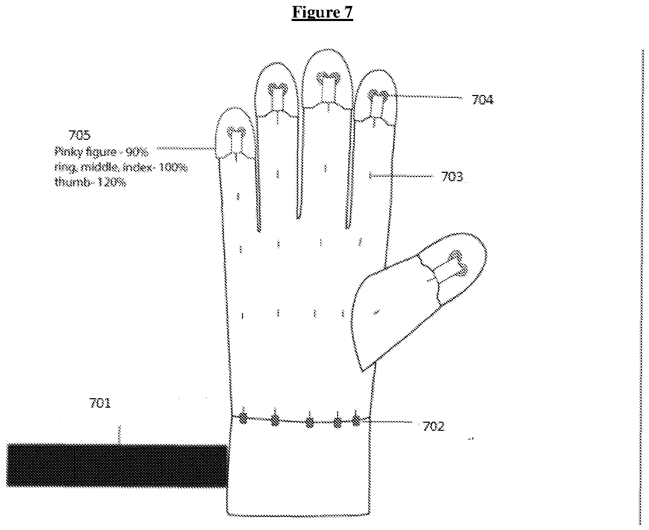

In an embodiment, the sensor glove is useful to detect the Range of Motion of the affected limb when the patients/ user come for follow up visits. In such embodiment, the patient can wear this glove on their affected hand and their progress in Range of motion can be remotely monitored using the Android Application for evaluating the improvement in the patient. **Referring to** **Figure 7****,** which illustrates front side of the motorized glove (2) showing Velcro straps (701) (11 inches length and 2.5 inches width, 1 inch corresponds to 2,54 cm.), rhodium plated column barrel swivel clasps (702), string (703), metal hooks(704), finger tip caps (705).

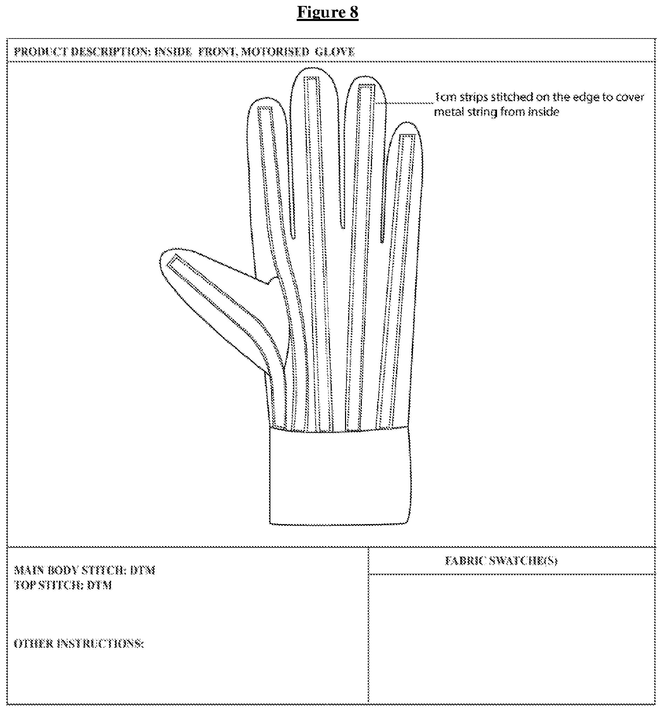

**Figure 8** illustrates inside layer front of the motorized glove (2) where 1 cm strips is stitched on the edge to cover metal string (703) from inside.

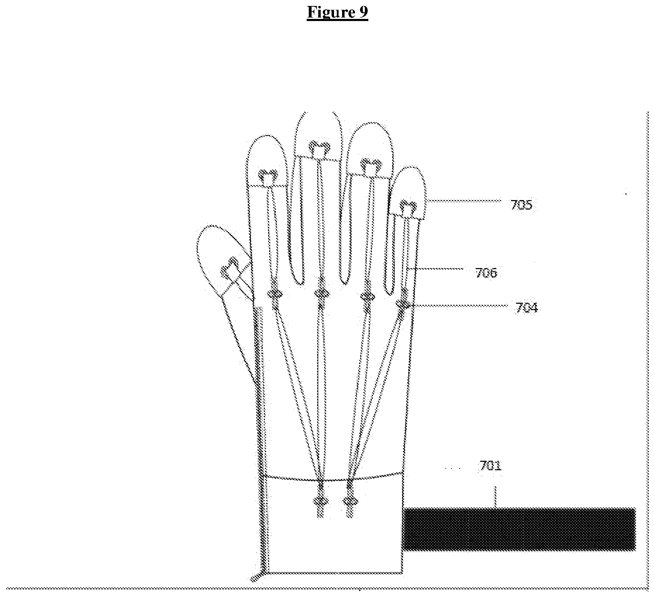

**Figure 9** illustrates back side of the motorized glove showing tensioner location and connections. The figure shows Velcro strap of approximately 11 inches, hooks (704) connecting through rubber band tensioners to finger tip cap (705) from back.

In another embodiment, the device comprises a force sensitive resistor on the fingertip to measure the Pinch force for evaluating the improvement in the patient.

In another embodiment, the motorized glove (2) comprises metal hooks (704) connected to the rhodium plated column barrel swivel clasps (702) via strings (703). The barrel swivel connectors act as the connecting point between glove and the motorized unit.

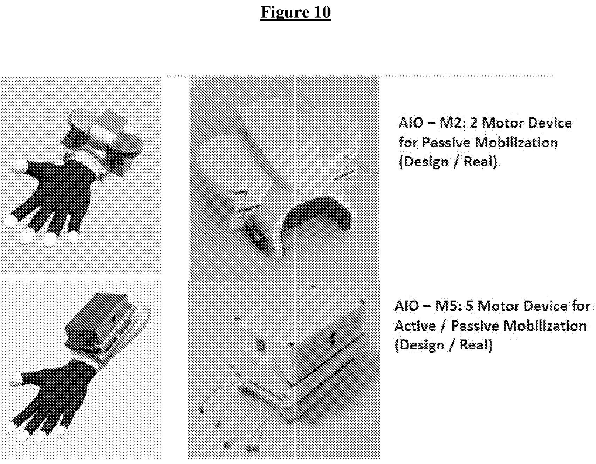

In an embodiment, the motorized unit (3) comprises 2 motors / 5 motors (allowing independent finger of each control) to be placed in the arm of the subject/ user/ patient, as illustrated in **Figure 10****.** The barrel swivel connectors (702) act as the connecting point between glove and the motorized unit (3). The unit also comprises microcontroller circuit (BLEI Wi-Fi), dedicated Motor Driver to power the motors. The unit is powered by a simple powerbank which can support the microcontroller at 3.3V and Motors at 5V simultaneously. The modular connectors help for choosing which finger to mobilize and activate active / passive mode of therapy based on requirement.

In an embodiment, the method of operation of the customizable and light-weight self-driven rehabilitation system with real time mirroring effect for mobilizing an affected/paralyzed hand of a user, as described above, comprises following steps:
a) wearing the sensor glove in the normal hand of a user followed by sensing the motion of the fingers by the sensor module wherein the sensor module sends the sensed/detected range of motion of finger to the control box for further transmission to the motorized glove through the communication module;
bi receiving the transmitted signal by the motorized unit, placed at the arm of the user, of the motorized glove, followed by the activation of the control unit configured to activate the drive unit to drive the plurality of motor/s adapted to mobilize the affected fingers connected with the tensioning wire through modular connector.

The closure unit/ mechanism is placed at the top of said motorized unit with a bridge in between. The closure unit/mechanism comprises the dial which allows the torque to be adjusted by its rotation for obtaining range of mobilization.

### Advantages of the Self driven rehabilitation device provided by the present invention:

a) The motorized glove is worn on the hand and the drive mechanism is placed on the arm. The user can easily place the arm and rest on any surface without affecting the motion.
b) The motorized glove is designed in such a way that it is modular and it can be used with any of the motorized systems.
c) The device is light-weight. When in air or surface, the load applied on the fingers will be only of the glove which is (50g). Hence, the device is super light-weight in comparison to the available products.
d) The device provides increased range of motion with two variable changes that account for change in range of mobilization as mentioned below:
   i) Moving the drive mechanism further back on the arm for increased range.
   ii) The Closure unit dial allows the torque to be adjusted by its rotation and setting the appropriate range of mobilization. This allows the position of the drive mechanism to be unchanged and still achieves the mobilization by varying the range of mobilization and torque.
e) Both Palm surfaces are free for movement and can move the wrist. If the wrist movement needs to be restricted, the design is made in such a way that it can accommodate a wrist splint under the glove while the drive mechanism allows maximum finger movement only.
f) The modular system includes 2 or 5 motors based on requirement so that the patient doesn't need to bear the extra weight. The connection is modular and is based on the clasp connector system. User can select which finger to mobilize while connecting the clasps.
g) Wrist splint can be added to provide restriction to wrist movement.
h) The device does not just trick the brain, but causes real mobilization at the same time.
i) The device is highly cost effective

The Invention is further described with the help of non-limiting examples:

### Example 1:

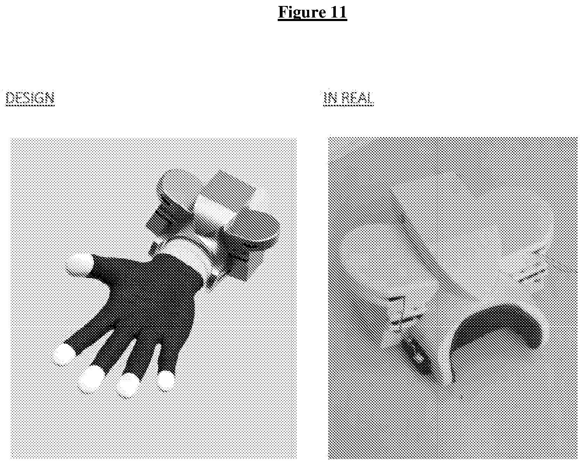

The device, as illustrated in **figure 11****,** is a two-motor device having modular nature and can operate in Various Modes as mentioned below:
Pinch Mode (Thumb + Index or Thumb+ Middle, so on)
Two Fingers connected to one motor for passive mobilization. (So, we can connect (Thumb + Middle +Ring + Little finger) for passive mode to mobilize for Grasp mode.
Single finger Flexion/ Extension. (Active / Passive)
Selected two finger mobilization. (Active / Passive)

### Example 2:

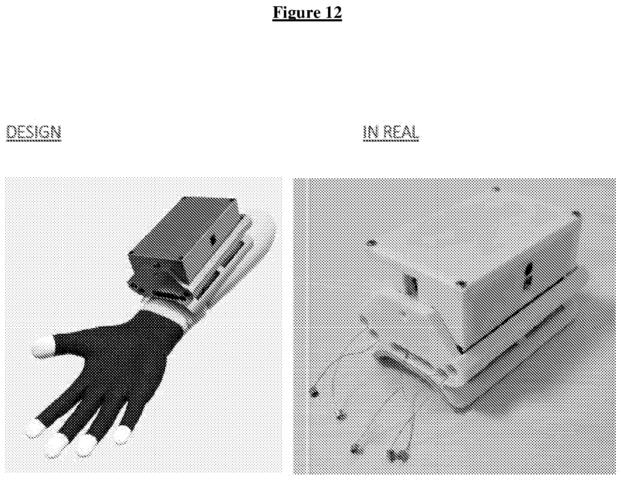

The device, as illustrated in **figure 12****,** is a five-motor device and can operate in various modes based on the requirement by the doctor as every patient has a different requirement of mobilization. Hence the patient/user can train/operate his normal hand in the way his affected hand should, and the patient can carry out the activity at home and vary it based on his improvement over time. The device helps in allowing independent movement of each fingers. The device helps in 13 modes of operation (PASSIVE - Pre Programmed) as following:
a. All open all dose
b. Counting exercise
c. Fist exercise
d. Grasping object exercise
e. Handle exercise
f. Picking objects exercise se
g. Pinch exercise
h. Pinches sequence exercise
l. Random numbers exercise
J. Single singer random exercise
k. Single finger sequence
l. Wave exercise.
m. Custom exercise for patient.

### Example 3:

The five Motor device comprises following components:
a) The barrel swivel connectors act as the connecting point between glove and the motorized unit
b) 5 Motors: (Independent control of each finger), Microcontroller circuit (BLEI Wi-Fi), Dedicated Motor Driver to power the motors
c) Powered by a simple Powerbank which can support the microcontroller at 3.3V and Motors at 5V simultaneously
d) Modular connectors help for choosing which finger to mobilize
e) Active / Passive mode of therapy is available based on requirement
f) Best used for nerve injury cases for better functional recovery
g) Remote monitoring and control.

### Example 4:

The motor device helps in improving range of motion m tendon injury patients and preventing stiffness arising due to non-compliance to therapy recommended to be done at harne.

The two-motor device comprises following components:
a) 2 motors. This can be connected to 4 fingers simultaneously (2 each) or the fingers can be connected based on Mode chosen. For example: 10 Modes + Cutsom Mode available to tailor make it to suit each patient need, Pinch Mode (Thumb + Index finger)
b) Light-weight, half the weight of five motor device (only 220g)
c) Remote monitoring and control.

## Claims

1. A customizable and light weight self-driven rehabilitation device with real time mirroring effect configured for mobilizing an affected or paralyzed hand of a user comprising:
a pair of gloves comprising at least a sensor glove (1), adapted to be worn in the normal functioning hand of the user, and a modular motorized glove (2), adapted to be worn in said paralyzed hand of the user;
wherein said sensor (1) glove is connected with the modular motorized glove (2) through a communication module for active and/or passive mobilization of the paralyzed hand through real time mirroring effect;
wherein sensor glove, provided with an easy to slide system for the fingers, comprises:
A. at least a sensing module comprising plurality of flex sensors (401) for sensing movement of fingers based on the change in resistance;
B. a control or circuit box comprising a communication module for collecting the data from sensing module and transmitting to the motorized glove;
C. clincher connectors (402) connects the sensing module with the control or circuit box; and
D. a filter circuit (403) adapted to remove the noise signal between the sensing module and control box;
E. a powered supply means comprising a battery configured for powering the sensor module and control module;
wherein the modular motorized glove, which is provided with a drive mechanism adapted to be positioned on the arm of a user, comprises:
A. plurality of fingertip caps (705), configured to be worn on to the finger tips, attached to a tensioning string;
B. motorized unit, configured to be placed in the arm of user, comprising:
i) plurality of motors for allowing the independent control of the fingers of the paralyzed hand wherein each motor is connected to individual strings (703) for extension;
ii) a motor driver unit and mechanism to drive said plurality of motors wherein the driver unit and mechanism is adapted to move towards back on the arm for increased range of motion, wherein the motor driver unit andmechanism comprises 2 motor or 5 motor operation system, wherein the 2 motor operation system operates in plurality of active and /or passive mode by connecting at least two fingers of the hand or its combination thereof while the 5 motor operation system operate/s in plurality of modes based on the requirement of mobilization wherein the 5 motor operation system allows independent movement of each finger facilitating plurality of modes of operation for active /passive mode;
iii) a microcontroller unit with communication module configured to receive the data transmitted from said sensor glove to accordingly control the said motorized unit;
wherein the strings (703) are connected through the modular connector acting like a bridge between the motorized unit and glove;
iv) at least a closure unit and mechanism being placed at the top of said motorized unit with a bridge in between, said closure unit and mechanism comprises the characterizing feature of a dial which allows the torque to be adjusted by its rotation for obtaining range of mobilization;
v) a power supply means to power the motor driver unit and the microcontroller unit;
vii) connecting means comprising modular connector for selecting the finger to be mobilized and for the activation of active and/or passive mode of therapy based on requirement;
where said system optionally comprises wrist splint for restricting the wrist movement, **characterized in that** the modular motorized glove comprises a plurality of metal hooks (704) connected to a rhodium plated column comprising barrel swivel clasp connectors (702) wherein the barrel swivel clasp connectors connect the glove to the motorized unit.

2. The device as claimed in claim 1, wherein the motor operation system comprises a servo motor system.

3. The device as claimed in claim 1, wherein the bridge in the closure unit comprises a servo horn attachment.

4. The device as claimed in claim 1, wherein the sensor glove comprises a plurality of layers wherein the layers comprise:
a) a first layer, configured to consist of a plurality of Velcro strips (701) for binding the gloves and components that come in contact with the hand of the subject oruser;
b) an intermediate layer configured to in-house the sensor and connecting means comprising clincher connector; and
c) a third layer configured to position the sensor module and the clincher connectors.

5. The device as claimed in claim 1, wherein the sensor glove being in communication to the motorized glove provides feedback based on virtual reality environment.

6. The device as claimed in claim 1, wherein the sensor glove is configured to function in modes comprising:
a. active mode for transmitting position data for mobilizing the fingers;
b. diagnostic mode for wirelessly tracking the range of motion through a software application;
c. virtual reality gaming mode where the sensor glove tracks the range of motion and wirelessly transmits the data to a software application.

7. The device as claimed in claim 1, wherein said glove comprises grooves for the strings configured to slide through with ease for providing maximum torque.

8. The device as claimed in claim 1, wherein the range of motion of the affected limb is configured to be remotely monitored by an application software for evaluating the improvement in the user.

9. The device as claimed in a claim 1, wherein the clincher connectors comprise plastic coating configured for eliminating the overlap of signals received from the plurality of sensors.

10. The device as claimed in claim 1, wherein the modular connector of motorized glove is in connection to the Flexible fingertip cap where the fingertip cap also attaches itself to the tensioning system on the back side configured to provide passive extension or passive flexion.

11. The device as claimed in a claim 1, wherein the sensor glove comprises a force sensitive resistor on the fingertip configured to measure the pinch force for evaluating the improvement in the patient.

## Patentansprüche

1. Ein anpassbares und leichtes selbstfahrendes Rehabilitationsgerät mit Echtzeit-Spiegeleffekt, das zur Mobilisierung einer betroffenen oder gelähmten Hand eines Benutzers konfiguriert ist, umfassend:
ein Paar Handschuhe, bestehend aus mindestens einem Sensorhandschuh (1), der an der normal funktionierenden Hand des Benutzers getragen wird, und einem modularen motorisierten Handschuh (2), der an der gelähmten Hand des Benutzers getragen wird;
wobei der Sensorhandschuh (1) über ein Kommunikationsmodul mit dem modularen motorisierten Handschuh (2) verbunden ist, um die gelähmte Hand durch einen Echtzeit-Spiegeleffekt aktiv und/oder passiv zu mobilisieren;
wobei der Sensorhandschuh, der mit einem leicht gleitenden System für die Finger versehen ist, umfasst:
A. mindestens ein Sensormodul mit mehreren Biegesensoren (401) zum Erfassen der Bewegung der Finger auf der Grundlage der Änderung des Widerstands;
B. eine Steuer- oder Schaltbox, die ein Kommunikationsmodul zum Sammeln der Daten aus dem Sensormodul und zum Übertragen an den motorisierten Handschuh umfasst;
C. Clincher-Steckverbinder (402), die das Sensormodul mit der Steuer- oder Schaltbox verbinden; und
D. eine Filterschaltung (403), die dazu ausgelegt ist, das Rauschsignal zwischen dem Sensormodul und der Steuerbox zu entfernen;
E. eine Stromversorgungseinrichtung, die eine Batterie umfasst, die zum Versorgen des Sensormoduls und des Steuermoduls mit Strom konfiguriert ist;
wobei der modulare motorisierte Handschuh, der mit einem Antriebsmechanismus versehen ist, der dazu ausgelegt ist, am Arm eines Benutzers positioniert zu werden, umfasst:
A. eine Vielzahl von Fingerspitzenkappen (705), die zum Tragen auf den Fingerspitzen konfiguriert sind und an einer Spannschnur befestigt sind;
B. eine motorisierte Einheit, die so konfiguriert ist, dass sie am Arm des Benutzers angebracht werden kann, und Folgendes umfasst:
i) eine Vielzahl von Motoren, die eine unabhängige Steuerung der Finger der gelähmten Hand ermöglichen, wobei jeder Motor mit einzelnen Schnüren (703) zur Streckung verbunden ist;
ii) eine Motortreibereinheit und einen Mechanismus zum Antreiben der mehreren Motoren, wobei die Treibereinheit und der Mechanismus so ausgelegt sind, dass sie sich für einen größeren Bewegungsbereich nach hinten auf dem Arm bewegen können, wobei die Motortreibereinheit und der Mechanismus ein 2-Motor- oder 5-Motor-Betriebssystem umfassen, wobei das 2-Motor- Betriebssystem in mehreren aktiven und/oder passiven Modi arbeitet, indem es mindestens zwei Finger der Hand oder eine Kombination davon verbindet, während das 5-Motor-Betriebssystem in mehreren Modi basierend auf den Mobilisierungsanforderungen arbeitet, wobei das 5-MotorBetriebssystem eine unabhängige Bewegung jedes Fingers ermöglicht, was mehrere Betriebsmodi für den aktiven/passiven Modus ermöglicht;
iii) eine Mikrocontroller-Einheit mit einem Kommunikationsmodul, das so konfiguriert ist, dass es die von dem Sensorhandschuh übertragenen Daten empfängt, um die motorisierte Einheit entsprechend zu steuern;
wobei die Schnüre (703) über den modularen Verbinder verbunden sind, der wie eine Brücke zwischen der motorisierten Einheit und dem Handschuh wirkt;
iv) mindestens eine Verschlusseinheit und ein Mechanismus, die oben auf der motorisierten Einheit mit einer Brücke dazwischen angeordnet sind, wobei die Verschlusseinheit und der Mechanismus das charakteristische Merkmal eines Drehknopfs aufweisen, der es ermöglicht, das Drehmoment durch Drehen einzustellen, um den Bewegungsbereich zu erhalten;
v) eine Stromversorgungseinrichtung zum Versorgen der Motortreibereinheit und der Mikrocontroller-Einheit mit Strom;
vi) Verbindungsmittel, umfassend einen modularen Verbinder zur Auswahl des zu bewegenden Fingers und zur Aktivierung des aktiven und/oder passiven Therapiemodus je nach Bedarf;
wobei das System optional eine Handgelenkschiene zur Einschränkung der Handgelenksbewegung umfasst, **dadurch gekennzeichnet, dass** der modulare motorisierte Handschuh eine Vielzahl von Metallhaken (704) umfasst, die mit einer rhodinierten Säule verbunden sind, die drehbare Fassverschlussverbinder (702) umfasst, wobei die drehbaren Fassverschlussverbinder den Handschuh mit der motorisierten Einheit verbinden.

2. Vorrichtung nach Anspruch 1, wobei das Motorbetriebssystem ein Servomotorsystem umfasst.

3. Vorrichtung nach Anspruch 1, wobei die Brücke in der Verschlusseinheit eine Servohornbefestigung umfasst.

4. Die Vorrichtung nach Anspruch 1, wobei der Sensorhandschuh mehrere Schichten umfasst, wobei die Schichten Folgendes umfassen:
a) eine erste Schicht, die so konfiguriert ist, dass sie aus einer Vielzahl von Klettverschlussstreifen (701) besteht, um die Handschuhe und Komponenten, die mit der Hand des Probanden oder Benutzers in Kontakt kommen, zu befestigen;
b) eine Zwischenschicht, die so konfiguriert ist, dass sie den Sensor und Verbindungsmittel umfasst, die einen Clincher-Verbinder umfassen; und
c) eine dritte Schicht, die so konfiguriert ist, dass sie das Sensormodul und die Clincher-Verbinder positioniert.

5. Die Vorrichtung gemäß Anspruch 1, wobei der Sensorhandschuh, der mit dem motorisierten Handschuh in Verbindung steht, ein Feedback auf der Grundlage einer virtuellen Realität liefert.

6. Die Vorrichtung nach Anspruch 1, wobei der Sensorhandschuh so konfiguriert ist, dass er in Modi funktioniert, die Folgendes umfassen:
a. Aktivmodus zum Übertragen von Positionsdaten für die Mobilisierung der Finger;
b. Diagnosemodus zur drahtlosen Verfolgung des Bewegungsumfangs über eine Softwareanwendung;
c. Virtual-Reality-Spielmodus, in dem der Sensorhandschuh den Bewegungsbereich verfolgt und die Daten drahtlos an eine Softwareanwendung überträgt.

7. Das Gerät gemäß Anspruch 1, wobei der Handschuh Rillen für die Schnüre umfasst, die so konfiguriert sind, dass sie leicht gleiten können, um ein maximales Drehmoment zu erzielen.

8. Das Gerät gemäß Anspruch 1, wobei der Bewegungsbereich des betroffenen Gliedes so konfiguriert ist, dass er durch eine Anwendungssoftware ferngesteuert überwacht werden kann, um die Verbesserung beim Benutzer zu bewerten.

9. Das Gerät gemäß Anspruch 1, wobei die Clincher-Verbinder eine Kunststoffbeschichtung aufweisen, die so konfiguriert ist, dass sie die Überlagerung der von den mehreren Sensoren empfangenen Signale eliminiert.

10. Vorrichtung nach Anspruch 1, wobei der modulare Verbinder des motorisierten Handschuhs mit der flexiblen Fingerspitzenkappe verbunden ist, wobei die Fingerspitzenkappe ebenfalls an dem Spannsystem auf der Rückseite befestigt ist, das so konfiguriert ist, dass es eine passive Streckung oder passive Beugung ermöglicht.

11. Das Gerät gemäß Anspruch 1, wobei der Sensorhandschuh einen kraftempfindlichen Widerstand an der Fingerspitze umfasst, der so konfiguriert ist, dass er die Klemmkraft misst, um die Verbesserung beim Patienten zu bewerten.

## Revendications

1. Dispositif de rééducation personnalisable et léger, à entraînement autonome, avec effet miroir en temps réel, configuré pour mobiliser une main affectée ou paralysée d'un utilisateur, comprenant :
une paire de gants comprenant au moins un gant capteur (1), adapté pour être porté sur la main fonctionnelle de l'utilisateur, et un gant motorisé modulaire (2), adapté pour être porté sur ladite main paralysée de l'utilisateur;
dans lequel ledit gant capteur (1) est connecté au gant motorisé modulaire (2) par l'intermédiaire d'un module de communication pour la mobilisation active et/ou passive de la main paralysée grâce à un effet miroir en temps réel;
dans lequel le gant capteur, muni d'un système facile à faire glisser pour les doigts, comprend :
A. au moins un module de détection comprenant une pluralité de capteurs flexibles (401) pour détecter le mouvement des doigts en fonction du changement de résistance;
B. un boîtier de commande ou de circuit comprenant un module de communication pour collecter les données du module de détection et les transmettre au gant motorisé;
C. des connecteurs clincher (402) reliant le module de détection au boîtier de commande ou au boîtier de circuit; et
D. un circuit de filtrage (403) adapté pour supprimer le signal de bruit entre le module de détection et le boîtier de commande;
E. un moyen d'alimentation électrique comprenant une batterie configurée pour alimenter le module capteur et le module de commande;
dans lequel le gant motorisé modulaire, qui est muni d'un mécanisme d'entraînement adapté pour être positionné sur le bras d'un utilisateur, comprend:
A. une pluralité de capuchons pour doigts (705), configurés pour être portés sur le bout des doigts, fixés à un cordon de tension ;
B. une unité motorisée, configurée pour être placée dans le bras de l'utilisateur, comprenant :
i) une pluralité de moteurs permettant le contrôle indépendant des doigts de la main paralysée, chaque moteur étant relié à des cordes individuelles (703) pour l'extension;
ii) une unité d'entraînement de moteur et un mécanisme pour entraîner ladite pluralité de moteurs, dans laquelle l'unité d'entraînement et le mécanisme sont adaptés pour se déplacer vers l'arrière sur le bras afin d'augmenter l'amplitude de mouvement, dans lequel l'unité d'entraînement du moteur et le mécanisme comprennent un système de fonctionnement à 2 moteurs ou à 5 moteurs, dans lequel le système de fonctionnement à 2 moteurs fonctionne dans une pluralité de modes actifs et/ou passifs en connectant au moins deux doigts de la main ou une combinaison de ceux-ci, tandis que le système de fonctionnement à 5 moteurs fonctionne dans une pluralité de modes en fonction des besoins de mobilisation, dans lequel le système de fonctionnement à 5 moteurs permet un mouvement indépendant de chaque doigt, facilitant une pluralité de modes de fonctionnement pour le mode actif/passif;
iii) une unité de microcontrôleur avec un module de communication configuré pour recevoir les données transmises par ledit gant capteur afin de contrôler en conséquence ladite unité motorisée; dans lequel les cordons (703) sont connectés par l'intermédiaire du connecteur modulaire agissant comme un pont entre l'unité motorisée et le gant;
iv) au moins une unité de fermeture et un mécanisme placés au sommet de ladite unité motorisée avec un pont entre les deux, ladite unité de fermeture et ledit mécanisme comprenant la caractéristique distinctive d'un cadran qui permet de régler le couple par sa rotation afin d'obtenir une plage de mobilisation;
v) un moyen d'alimentation électrique pour alimenter l'unité d'entraînement du moteur et l'unité de microcontrôleur;
vi) moyens de connexion comprenant un connecteur modulaire pour sélectionner le doigt à mobiliser et pour activer le mode actif et/ou passif de la thérapie en fonction des besoins;
ledit système comprenant éventuellement une attelle de poignet pour limiter le mouvement du poignet, **caractérisé en ce que** le gant motorisé modulaire comprend une pluralité de crochets métalliques (704) reliés à une colonne plaquée rhodium comprenant des connecteurs à fermoir pivotant en forme de barillet (702), les connecteurs à fermoir pivotant en forme de barillet reliant le gant à l'unité motorisée.

2. Dispositif selon la revendication 1, dans lequel le système de fonctionnement du moteur comprend un système de servomoteur.

3. Dispositif selon la revendication 1, dans lequel le pont dans l'unité de fermeture comprend une fixation de servo-corne.

4. Dispositif selon la revendication 1, dans lequel le gant à capteur comprend une pluralité de couches, les couches comprenant :
a) une première couche, configurée pour être constituée d'une pluralité de bandes Velcro (701) pour attacher les gants et les composants qui entrent en contact avec la main du sujet ou de l'utilisateur;
b) une couche intermédiaire configurée pour loger le capteur et des moyens de connexion comprenant un connecteur à sertir; et
c) une troisième couche configurée pour positionner le module capteur et les connecteurs à sertir.

5. Le dispositif selon la revendication 1, dans lequel le gant capteur étant en communication avec le gant motorisé fournit un retour d'information basé sur un environnement de réalité virtuelle.

6. Le dispositif selon la revendication 1, dans lequel le gant capteur est configuré pour fonctionner dans des modes comprenant :
a. mode actif pour transmettre des données de position afin de mobiliser les doigts;
b. mode diagnostic pour suivre sans fil l'amplitude de mouvement via une application logicielle;
c. mode jeu en réalité virtuelle dans lequel le gant à capteurs suit l'amplitude de mouvement et transmet sans fil les données à une application logicielle.

7. Dispositif selon la revendication 1, dans lequel ledit gant comprend des rainures pour les cordes, configurées pour glisser facilement afin de fournir un couple maximal.

8. Dispositif selon la revendication 1, dans lequel l'amplitude de mouvement du membre affecté est configurée pour être surveillée à distance par un logiciel d'application afin d'évaluer l'amélioration chez l'utilisateur.

9. Dispositif selon la revendication 1, dans lequel les connecteurs de serrage comprennent un revêtement en plastique configuré pour éliminer le chevauchement des signaux reçus de la pluralité de capteurs.

10. Dispositif selon la revendication 1, dans lequel le connecteur modulaire du gant motorisé est relié à l'embout flexible pour doigt, lequel embout se fixe également au système de tension situé à l'arrière et configuré pour fournir une extension passive ou une flexion passive.

11. Dispositif selon la revendication 1, dans lequel le gant à capteurs comprend une résistance sensible à la force sur le bout du doigt, configurée pour mesurer la force de pincement afin d'évaluer l'amélioration chez le patient.
